# EUROPEAN PATENT APPLICATION

(11) **EP 3 928 754 A1**
(43) Date of publication of application: **29.12.2021**
(21) Application number: 20182931.4
(22) Date of filing: 29.06.2020
(51) Int. Cl.: A61F 13/475, A61F 13/49, A61F 13/532, A61F 13/533, A61F 13/534

(54) **ABSORBENT ARTICLES WITH CHANNELED ABSORBENT CORES**

(30) Priority: 24.06.2020 US 202063043361 P
(71) Applicant: Associated Hygienic Products LLC, Duluth, Georgia 30096 (US)
(72) Inventor: Kaiser, Thomas A., Marion, Ohio 43302 (US); Ottery, Trenton T., Delaware, Ohio 43015-8780 (US)
(74) Representative: Lohr, Jöstingmeier & Partner

(57) **Abstract**

The present disclosure describes absorbent articles that include an absorbent core. The absorbent core extends longitudinally along a crotch portion of the absorbent article. The absorbent core includes a channel having one or more pathways extending longitudinally along the absorbent core. The channel may be substantially free of absorbent material. In some implementations, the channel includes a single pathway. In other implementations, the channel includes multiple pathways.

## Description

### FIELD OF DISCLOSURE

The present invention relates generally to absorbent products like adult incontinence briefs, protective underwear, feminine hygiene pads, and infant diapers, youth pants, training pants, and the like; and more particularly, but not by way of limitation, to absorbent articles having absorbent cores with channels that are substantially free of absorbent material.

### BACKGROUND

Absorbent products can include, for example, disposable absorbent articles that are wearable by a user, examples of which include baby diapers, training pants, and adult incontinence briefs and underwear, all of which may be made in disposable forms. "Disposable" refers to articles that are designed to be discarded after a limited use rather than being laundered or otherwise restored for reuse. Disposable absorbent products have met with widespread acceptance in the marketplace for a variety of applications, including infant and adult incontinence care, in view of the manner in which such products can provide effective and convenient liquid absorption and retention while maintaining the comfort of the wearer. Such disposable absorbent articles often include a topsheet that is configured to be closest to the wearer during use, a liquid-impermeable backsheet or outer cover, and an absorbent core located between the topsheet and the backsheet. In some instances, such disposable absorbent articles also include an acquisition-distribution layer (ADL) disposed between the topsheet and the absorbent core, in addition to other layers for distribution containment, reinforcement, etc., that may be located above or below the absorbent core. Elasticated standing leg cuffs and leg gathers are also often used in such articles to provide improved fit and reduced leakage around a wearer's legs, relative to articles without such cuffs or gathers.

U.S. Patent No. 4,670,011 discloses certain prior art examples of diapers, and U.S. Patents No. 6,976,978 and No. 4,940,464 disclose certain prior art examples of disposable incontinence garments or training pants.

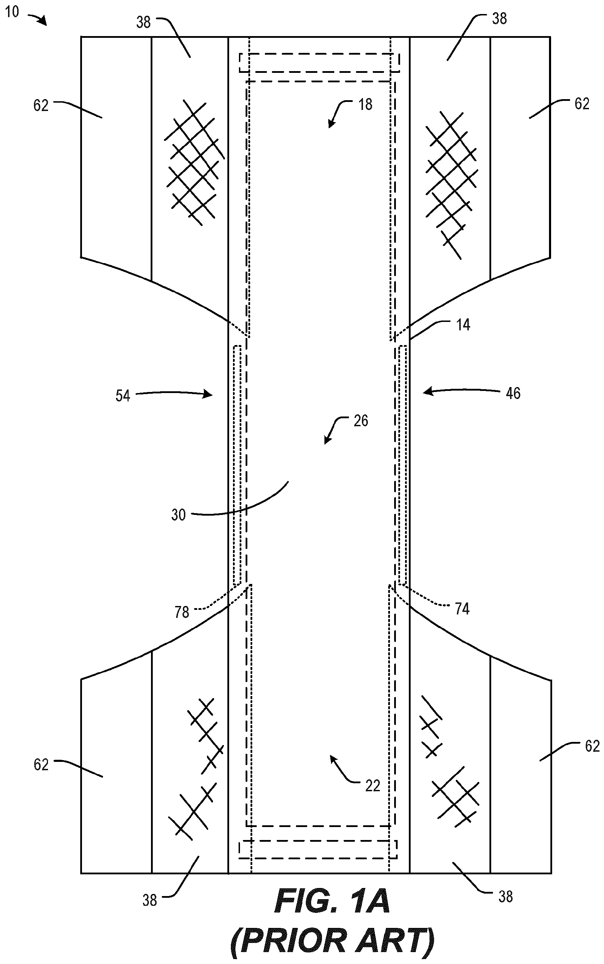

One example of such a disposable absorbent article is shown in FIGS. 1A-1B, which depict a lower plan view and a perspective view, respectively, of adult protective underwear 10. Underwear 10 includes a chassis 14 having a front waist portion 18, an opposing rear waist portion 22, and a crotch portion 26 extending longitudinally between front and rear waist portions 18, 22. Chassis 14 further includes a backsheet 30 defining an outer surface and configured to face away from a wearer during use of the underwear, and topsheet 34 defining an opposing body facing surface and configured to face a wearer during use of the underwear.

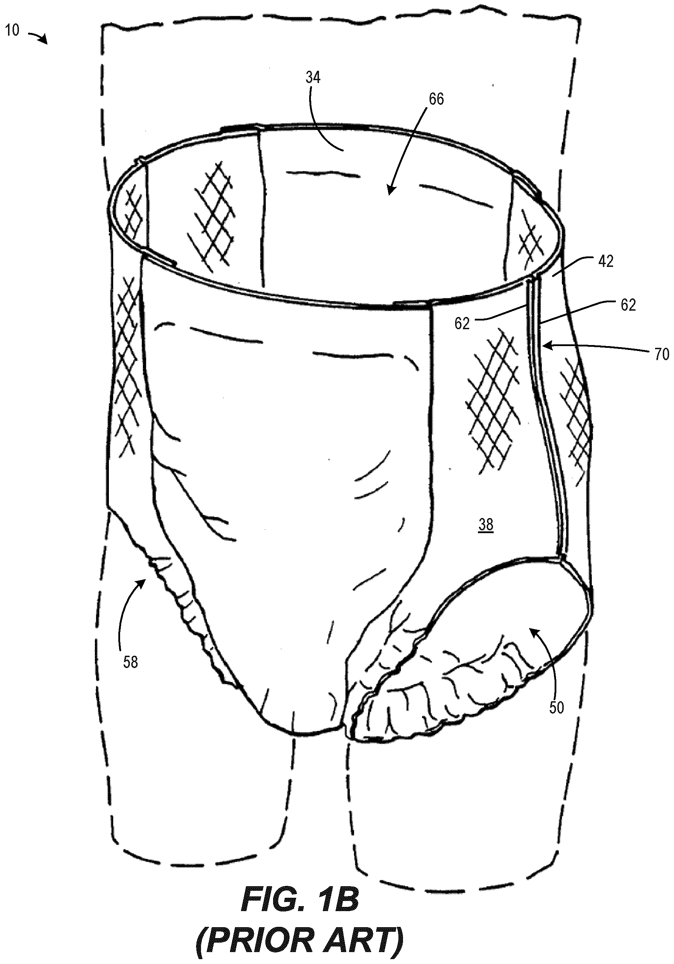

As shown in FIGS. 1A-1B, underwear 10 further includes a pair of front elastic side panels 38 and a pair of rear elastic side panels 42 configured to couple rear waist portion 22 to front waist portion 18 in a well-known configuration in which a left side 46 of the chassis defines a first leg opening 50 for a wearer's left leg, and in which a right side 54 of the chassis defines a second leg opening 58 for the wearer's right leg. In the depicted configuration, each of side panels 38, 42 includes a connection portion 62 configured to be coupled to a connection portion 62 of another of side panels 38, 42. Specifically, connection portion 62 of the left one of front side panels 38 is configured to be coupled to connection portion 62 of the left one of rear side panels 42, and connection portion 62 of the right one of front side panels 38 is configured to be coupled to connection portion 62 of the right one of rear side panels 42, such that the waist portions 18, 22 and side panels, 38, 42 cooperate to define a waist opening 66 as shown in FIG. 1B. Connection portions 62 of the respective side panels can be permanently coupled together to define a tear-able side seam 70, such as, for example, via adhesive, ultrasonic, or thermal bonds. Such tear-able side seams generally cannot be refastened, and thereby render an article unusable once opened. Alternatively, connection portions 62 of the respective side panels can be removably coupled to define a refastenable or adjustable side seam, such as, for example, via hook-and-loop fasteners. Hook and loop fasteners are mechanical fasteners that include hooks, such as in a hook fastener portion, that are configured to engage loops in a loop fastener portion or in fibers of a sheet of fabric; for example, a nonwoven or woven fabric with fibers that define open or loop-like regions into which the hooks can extend and engage. Examples of such hook and loop fasteners may be referred to as VELCRO.

As is known in the art, underwear 10 can include one or more elastic elements coupled to the chassis such that the one or more elastic elements resist expansion of a circumference of the first leg opening and resist expansion of a circumference of the second leg opening. For example, as shown in FIG. 1A, the depicted embodiment of the chassis (14) includes a first elastic region 74 along left side 46, and a second elastic region 78 along right side 54. In some configurations, elastic regions 74, 78 can each be defined by one or more elastic strands, which may be referred to in the art as "leg elastics," coupled to the chassis, for example laminated between the topsheet or an additional leg cuff layer and the backsheet. In other configurations, elastic regions 74, 78 can each be defined by an elastic film coupled to the chassis, for example laminated between the topsheet and the backsheet. In configurations in which elastic regions 74, 78 are defined by elastic film, the regions can be defined by separate pieces of elastic film or by separate regions of a single piece of elastic film. As shown in FIG. 1A, elastic regions 74, 78 may be parallel to and/or extend along a majority of a length of each of sides 46 and 54, provided that the elastic regions are configured to provide a biasing force that resists expansion of the leg openings when the chassis is in its closed configuration and tends to contract the leg opening around a wearer's leg, as shown in FIG. 1B. Contraction of the leg opening to conform to the wearer's leg is desired for good containment of urine and feces in an absorbent product.

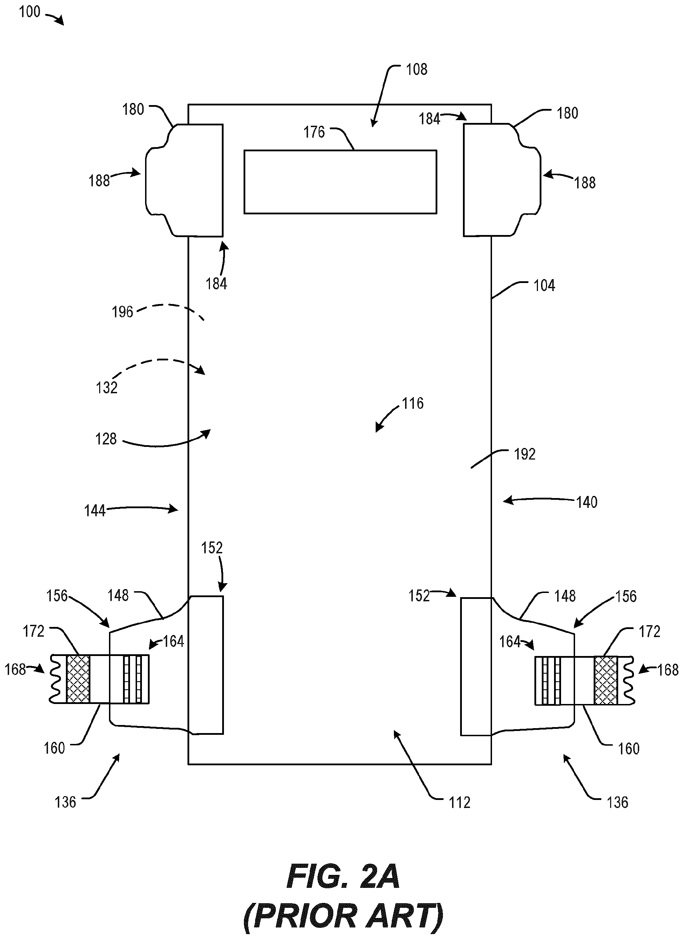

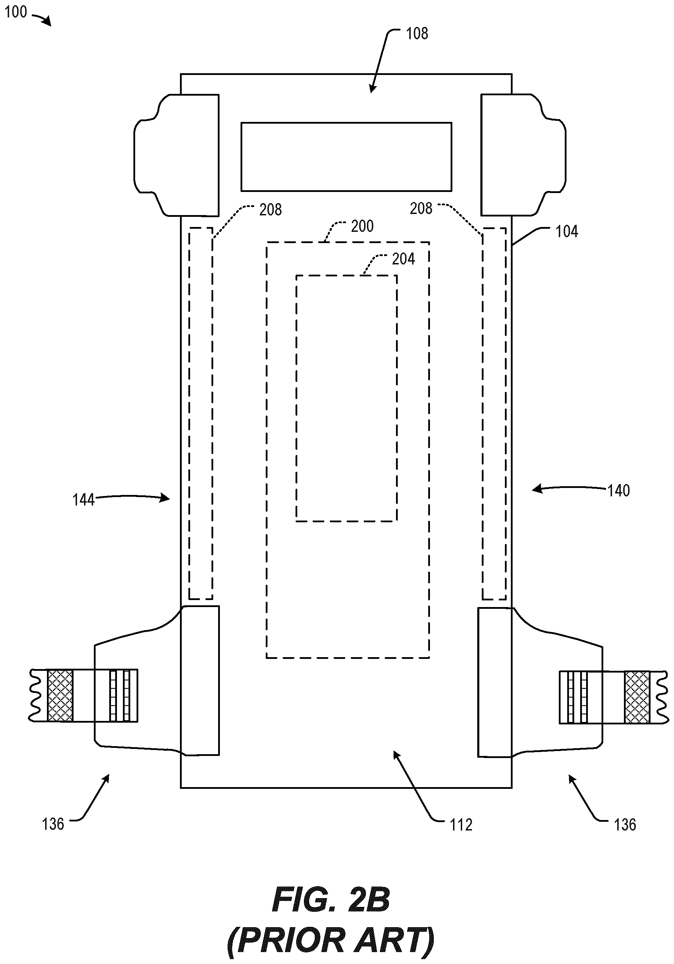

Another example of such a disposable absorbent article is shown in FIGS. 2A and 2B, which depict lower plan views of a baby diaper 100. Diaper 100 includes a chassis 104 having a front waist portion 108, an opposing rear waist portion 112, and a crotch portion 116 extending longitudinally between front and rear waist portions 108, 112. Chassis 104 further includes an outer surface 128 configured to face away from a wearer during use of the diaper, and an opposing body facing surface 132 configured to face a wearer during use of the diaper. In the view of FIG. 2A, a dashed leader extends from the body facing surface to reference numeral 132 because body facing surface 132 is opposite outer surface 128 and therefore not visible in the view of FIG. 2A.

As shown in FIG. 2A, diaper 100 further includes a pair of closure members 136 configured to couple rear waist portion 112 to front waist portion 108 in a well-known configuration in which a left side 140 of the chassis defines a first leg opening for a wearer's left leg, and in which a right side 144 of the chassis defines a second leg opening for the wearer's right leg, similar in some respects to what is shown in FIG. 1B for underwear 10 (or a training pant). In the depicted configuration, the closure members include a pair of back ears or back ear panels 148 each having a first end 152 bonded to rear waist portion 112 of chassis 104, and a second end 156 shown extending away from rear waist portion 112. "Bonded" refers to the joining, adhering, connecting, attaching, or the like, of two elements via adhesive(s), ultrasonic bond(s), and/or thermal bond(s). Two elements will be considered to be bonded together when they are bonded directly to one another or indirectly to one another, such as when each is directly bonded to intermediate elements.

Each closure member 136 further includes a fastener tab 160 with a first end 164 bonded to back ear 148, a second end 168 shown extending laterally outward from back ear 148, and a fastener portion 172 coupled to the fastener tab. Back ears 148 are each formed of a stretchable elastic material, such as a nonwoven laminate, that permit adjustment in the width and tension of back ears 148 to vary the form and fit of diaper 100 when worn by a user.

Fastener tabs 160 are formed of an inelastic nonwoven material and carry fastener portions 172. Fastener portions 172 can include strips of hook material configured to interact with a corresponding loop material in the well-known hook-and-loop fastener arrangement. Connection of closure members 136 to front waist portion 108 is facilitated by a landing zone 176 configured to be engaged by fastener portions 172. In this embodiment, landing zone 176 is defined by an anchoring member that includes a strip of loop material bonded to front waist portion 108 of chassis 104, for example, to the backsheet, and configured to be engaged by the hook material of fastener portions 172.

As shown in FIG. 2A, diaper 100 also includes a pair of front ears 180 extending from opposite sides 140, 144 of chassis 104 with each of front ears 180 each having a first end 184 bonded to front waist portion 108 of chassis 104, and a second end 188 shown extending away from a respective side of front waist portion 108. Front ears 180 are each formed of a relatively soft nonwoven material and are each configured for grasping by the caregiver during application of the diaper, as well as to be overlapped by the corresponding fastener tab 160 and/or back ear 148 to prevent the edges of fastener tab 160 from pinching, rubbing, or otherwise irritating a user's skin in use when fastening portions 172 are engaged with landing zone 176 to couple rear waist portion 112 to front waist portion 108. In some embodiments, front ears 180 include loop fastener portions or a fabric that is configured to be engaged by hook fastener portions such that fastener portions 172 can engage front ears 180.

Outer surface 128 is defined by a liquid-impermeable backsheet 192 that defines outer surface 128, and a liquid-permeable topsheet 196 that defines body facing surface 132 and is configured to be closest to the wearer during use. "Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. "Lamination" is the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are permanently assembled by heat, pressure, ultrasonic welding, or adhesives.

As shown in FIG. 2B, the depicted embodiment include an absorbent core 200 disposed between topsheet 196 and backsheet 192. An "absorbent core" is a structure typically disposed between a topsheet and backsheet of an absorbent article and containing materials like super absorbent particles (SAP) and/or cellulosic fibers that are configured to absorb liquid in the absorbent article. Absorbent core 200 may also be contained by a core cover that extends over the top and/or bottom sides of absorbent core 200 and may be continuous or sealed along the edges of absorbent core 200.

As shown in FIG. 2B, diaper 100 also includes an acquisition-distribution layer (ADL) 204 disposed between the topsheet and the absorbent core. "Layer" when used in the singular can be a single element or a plurality of elements. For example, a plurality of sheets may together define a single layer, such as, for example, a layer with a particular function to which the sheets of the layer contribute. Additional layers for fluid management, reinforcement, and performance enhancement may be incorporated inside the core cover or outside the core cover and between topsheet 196 and backsheet 192.

As is known in the art, diaper 100 can include one or more elastic elements coupled to the chassis such that the one or more elastic elements resist expansion of a circumference of the first leg opening and resist expansion of a circumference of the second leg opening. For example, as shown in FIG. 2B, the depicted configuration of the chassis (104) includes a first elastic region 208 along left side 140, and a second elastic region 208 along right side 144. In some configurations, elastic regions 208 can each be defined by one or more elastic strands, which may be referred to in the art as "leg elastics," coupled to the chassis, for example laminated between the topsheet (or an additional leg cuff layer) and the backsheet. In other configurations, elastic regions 208 can each be defined by an elastic film coupled to the chassis, for example laminated between the topsheet (or an additional leg cuff layer) and the backsheet. In configurations in which elastic regions 208 are defined by elastic film, the regions can be defined by separate pieces of elastic film or by separate regions of a single piece of elastic film. As shown in FIG. 2B, elastic regions 208 may be parallel to and/or extend along a majority of a length of each of sides 140 and 144, provided that the elastic regions are configured to provide a biasing force that resists expansion of the leg openings when the chassis is in its closed configuration.

Diaper 100 of FIGS. 2A and 2B is typically packaged and sold in a folded, and unfastened configuration in which chassis 104 is folded in half such that rear waist portion 112 overlaps front waist portion 108, but fastener portions 172 do not engage landing zone 176. While diaper 100 is described as a baby diaper, diaper 100 can also comprise an adult incontinence brief or youth training pant.

Some conventional absorbent articles use randomly dispersed SAP and fiber loosely bonded to the backsheet and the topsheet materials using adhesives or a similar type of air form construction placed within a core cover or an envelope. In the conventional absorbent articles, the absorbent core may not easily take a contoured shape of the user, causing fit issues. Additionally, materials shifted and bunched during use, which allowed absorbent materials to migrate within the absorbent core to areas outside or away from insult zones. Allowing the absorbent material to migrate away from the insult zones may compromise the effectiveness of the absorbent core at absorbing fluid insults.

### SUMMARY

The present disclosure is related to articles (e.g., absorbent articles) that that reduce the likelihood or prevent the migration of absorbent materials in an absorbent core through the use of channels. For example, an absorbent core can include a channel that extends longitudinally along the absorbent core. The channel may preferably be substantially free of absorbent material. Because the channel may preferably be substantially free of absorbent material, the channel may provide a pathway for channeling fluids longitudinally through the absorbent core. Additionally, a topsheet may be bonded to a backsheet or an upper core cover can be bonded to a lower core cover through the channel, which improves core integrity and helps hold absorbent materials in place during use of the absorbent core. The channel also helps to reduce crotch width reduction during use and assists in contouring the absorbent article to the wearer, improving the fit.

In some implementations, the channel may be a single pathway that extends longitudinally and may be configured to channel fluids longitudinally throughout the absorbent core. In other implementations, the channel may include multiple pathways to help channel fluids in both longitudinal and transverse directions, as further described and illustrated herein. In some implementations, the absorbent core may include embossing regions that surround the channel and extend at least partially longitudinally along the absorbent core. The embossing regions improve core integrity of the absorbent core. Additionally, the embossing regions can further assist in contouring the absorbent article to the user, improving the fit.

Some aspects of the present absorbent articles comprise a chassis preferably having opposing front and rear waist portions and a crotch portion extending longitudinally between the front and rear waist portions. In such embodiments, the crotch portion may be configured to conform about at least one of a wearer's groin area, perineum, and rear when the chassis may be configured in a wearable configuration. When in the wearable configuration, the front and rear waist portions cooperate to encircle and define a waist opening, a left side of the chassis defines a first leg opening, and a right side of the chassis defines a second leg opening. In such aspects, the absorbent articles may further comprise an absorbent core extending longitudinally along the crotch portion. In such aspects, the absorbent core includes a channel having one or more pathways extending longitudinally along the absorbent core. In such aspects, the channel may be substantially free of absorbent material. The absorbent core may comprise an assembly disposed between a top core cover and a bottom core cover. The absorbent core assembly may be disposed between a topsheet and a backsheet.

Some additional aspects of the present absorbent articles comprise a chassis having opposing front and rear waist portions and a crotch portion extending longitudinally between the front and rear waist portions. In such embodiments, the crotch portion may be configured to conform about at least one of a wearer's groin area, perineum, and rear when the chassis may be configured in a wearable configuration. When in the wearable configuration, the front and rear waist portions cooperate to encircle and define a waist opening, a left side of the chassis defines a first leg opening, and a right side of the chassis defines a second leg opening. In such aspects, the absorbent articles may further comprise an absorbent core extending longitudinally along the crotch portion. In such aspects, the absorbent core includes a channel having one or more pathways extending longitudinally along the absorbent core. In such aspects, the channel may be substantially free of absorbent material. The channel includes a first pathway extending in a first direction, a second pathway extending at least partially in a second direction that may be opposite to the first direction, and a third pathway extending at least partially in the third direction. The absorbent core may comprise an assembly disposed between a top core cover and a bottom core cover. The absorbent core assembly may be disposed between a topsheet and a backsheet.

Some additional aspects of the present absorbent articles comprise a chassis having opposing front and rear waist portions and a crotch portion extending longitudinally between the front and rear waist portions. In such embodiments, the crotch portion may be configured to conform about at least one of a wearer's groin area, perineum, and rear when the chassis may be configured in a wearable configuration. When in the wearable configuration, the front and rear waist portions preferably cooperateto encircle and define a waist opening, a left side of the chassis defines a first leg opening, and a right side of the chassis defines a second leg opening. In such aspects, the absorbent articles may further comprise an absorbent core extending longitudinally along the crotch portion. In such aspects, the absorbent core includes a channel having one or more pathways extending longitudinally along the absorbent core. In such aspects, the channel may be substantially free of absorbent material. The channel includes a first pathway, a second pathway, a third pathway, and a fourth pathway. The first pathway and the second pathway extend at least partially in a first direction, and the third pathway and the fourth pathway extend at least partially in a second direction that may be opposite to the first direction. The absorbent core comprises an assembly disposed between a top core cover and a bottom core cover. The absorbent core assembly may be disposed between a topsheet and a backsheet.

Some additional aspects of the present absorbent articles comprise a chassis having opposing front and rear waist portions and a crotch portion extending longitudinally between the front and rear waist portions. In such embodiments, the crotch portion may be configured to conform about at least one of a wearer's groin area, perineum, and rear when the chassis may be configured in a wearable configuration. When in the wearable configuration, the front and rear waist portions preferably cooperateto encircle and define a waist opening, a left side of the chassis defines a first leg opening, and a right side of the chassis defines a second leg opening. In such aspects, the absorbent articles may further comprise an absorbent core extending longitudinally along the crotch portion. In such aspects, the absorbent core includes a channel having one or more pathways extending longitudinally along the absorbent core. In such aspects, the channel may be substantially free of absorbent material. The channel includes a first elliptical pathway that extends in a first direction and a second elliptical pathway that extends in a second direction that may be opposite to the first direction. The absorbent core may comprise an assembly disposed between a top core cover and a bottom core cover. The absorbent core assembly may be disposed between a topsheet and a backsheet.

In some of the foregoing aspects, the channel includes a single channel. Additionally, or alternatively, the single channel includes a single pathway extending longitudinally along the absorbent core. Additionally or alternatively, the absorbent core further includes embossing regions that extend at least partially longitudinally along the absorbent core. In some such aspects, the channel may be disposed laterally between a first embossing region of the embossing regions and a second embossing region of the embossing regions. In some such aspects, the first embossing region and the second embossing region are concave with reference to the channel.

In some of the foregoing aspects, the single channel includes a first pathway extending in a first direction, a second pathway extending at least partially in a second direction that may be opposite to the first direction, and a third pathway extending at least partially in the second direction. Alternatively, the single channel may include a first pathway, a second pathway, a third pathway, and a fourth pathway. The first pathway and the second pathway extend at least partially in a first direction, and the third pathway and the fourth pathway extend at least partially in a second direction that may be opposite to the first direction. Alternatively, the single channel includes a first elliptical pathway that extends in a first direction and a second elliptical pathway that extends in a second direction that may be opposite to the first direction.

In some of the foregoing aspects, the absorbent article further includes a wetness indicator coupled to the backsheet. The wetness indicator corresponds to at least a portion of the channel. Additionally, or alternatively, the top core cover may be coupled to the bottom core cover at the channel. Alternatively, the absorbent article further includes an acquisition-distribution layer (ADL) that may be positioned above the absorbent core assembly and below the topsheet. In some such aspects, the absorbent article further includes a dryness layer positioned in conjunction with the ADL. Additionally, or alternatively, the absorbent article further includes a containment layer positioned below the absorbent core assembly and above the backsheet.

As used herein, various terminology may be for the purpose of describing particular implementations only and may be not intended to be limiting of implementations. For example, as used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). The term "coupled" may be defined as connected, although not necessarily directly, and not necessarily mechanically; two items that are "coupled" may be unitary with each other. The terms "a" and "an" are defined as one or more unless this disclosure explicitly requires otherwise. The term "substantially" may be defined as largely but not necessarily wholly what may be specified - and includes what may be specified; e.g., substantially 90 degrees includes 90 degrees and substantially parallel includes parallel - as understood by a person of ordinary skill in the art. In any disclosed embodiment, the term "substantially" may be substituted with "within [a percentage] of' what may be specified, where the percentage includes 0.1, 1, 5, and 10 percent; and the term "approximately" may be substituted with "within 10 percent of' what may be specified. The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or. The phrase "A, B, C, or a combination thereof' includes A alone, B alone, C alone, a combination of A and B, a combination of A and C, a combination of B and C, or a combination of A, B, and C.

The terms "comprise" and any form thereof such as "comprises" and "comprising," "have" and any form thereof such as "has" and "having," and "include" and any form thereof such as "includes" and "including" are open-ended linking verbs. As a result, an apparatus that "comprises," "has," or "includes" one or more elements possesses those one or more elements, butis not limited to possessing only those elements. Likewise, a method that "comprises," "has," or "includes" one or more steps possesses those one or more steps, but may be not limited to possessing only those one or more steps.

Any implementation of any of the apparatuses, systems, and methods can consist of or consist essentially of - rather than comprise/include/have - any of the described steps, elements, and/or features. Thus, in any of the claims, the term "consisting of' or "consisting essentially of' can be substituted for any of the open-ended linking verbs recited above, in order to change the scope of a given claim from what it would otherwise be using the open-ended linking verb. Additionally, it will be understood that the term "wherein" may be used interchangeably with "where."

Further, a device or system that is configured in a certain way is configured in at least that way, but it can also be configured in other ways than those specifically described. Aspects of one example may be applied to other examples, even though not described or illustrated, unless expressly prohibited by this disclosure or the nature of a particular example. Some details associated with the aspects described above and others are described below.

Some details associated with the aspects are described above, and others are described below. Other implementations, advantages, and features of the present disclosure will become apparent after review of the entire application, including the following sections: Brief Description of the Drawings, Detailed Description, and the Claims.

### BRIEF DESCRIPTION OF THE DRAWINGS

The following drawings illustrate by way of example and not limitation. For the sake of brevity and clarity, every feature of a given structure may be not always labeled in every figure in which that structure appears. Identical reference numbers do not necessarily indicate an identical structure. Rather, the same reference number may be used to indicate a similar feature or a feature with similar functionality, as may non-identical reference numbers.
**FIG. 1A** is a bottom plan view of a prior art disposable absorbent article, specifically adult protective underwear, in an open configuration.
**FIG. 1B** is a perspective view of the protective underwear of FIG. 1A in a closed configuration.
**FIG. 2A** is a bottom plan view of a prior art disposable absorbent article, specifically a baby diaper, in an open configuration.
**FIG. 2B** is a bottom plan view of the diaper of FIG. 2A, in an open configuration, showing certain internal components of the diaper.
**FIG. 3** is a bottom plan view of an example of an absorbent article that includes an absorbent core with a channel that is substantially free of absorbent material.
**FIG. 4** is a bottom plan view of another example of an absorbent article that includes an absorbent core with a channel that is substantially free of absorbent material.
**FIG. 5** is a bottom plan view of an example of another absorbent article that includes an absorbent core with a channel that is substantially free of absorbent material.
**FIG. 6** is a bottom plan view of another example of an absorbent article that includes an absorbent core with a channel that is substantially free of absorbent material.
**FIG. 7** is a bottom plan view of another example of an absorbent article that includes an absorbent core with a channel that is substantially free of absorbent material.
**FIG. 8** is a bottom plan view of another example of an absorbent article that includes an absorbent core with a channel that is substantially free of absorbent material and that includes an acquisition-distribution layer (ADL).

### DETAILED DESCRIPTION OF ILLUSTRATIVE ASPECTS

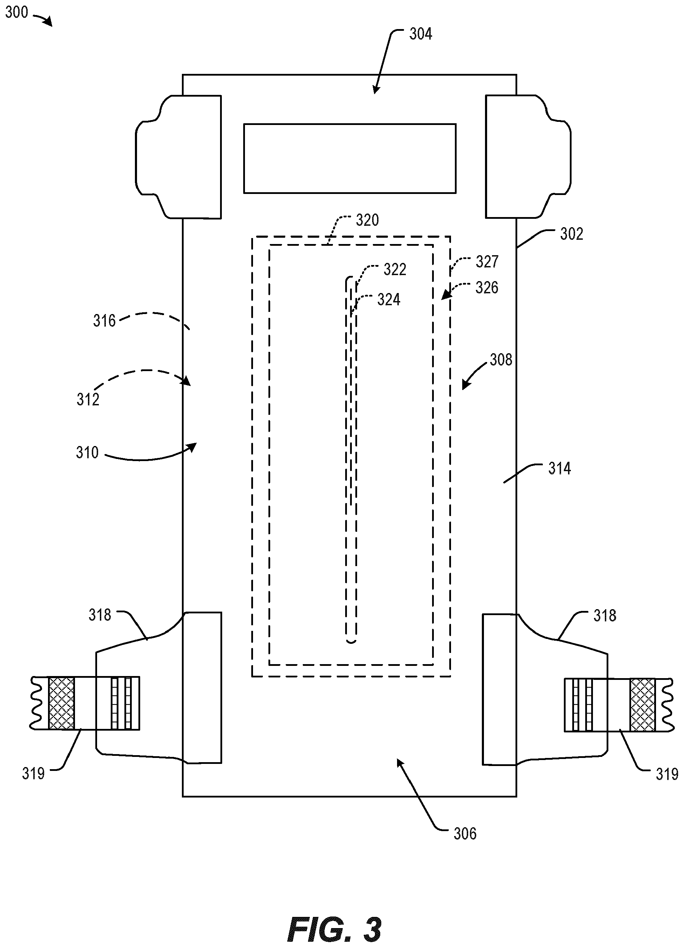

Referring to FIG. 3, an absorbent article 300 is shown. Absorbent article 300 may be a disposable absorbent article, such as adult incontinence briefs, protective underwear, feminine hygiene pads, infant diapers, youth training pants, and the like, as illustrative, non-limiting examples.

Absorbent article 300 includes a chassis 302 having a front waist portion 304, an opposing rear waist portion 306, and a crotch portion 308 extending longitudinally between front waist portion 304 and rear waist portion 306. Chassis 302 further includes an outer surface 310 configured to face away from a wearer during use of absorbent article 300, and an opposing body facing (or inner) surface 312 configured to face a wearer during use of absorbent article 300. In the view of FIG. 3, a dashed leader extends from the body facing surface to reference numeral 312 because body facing surface 312 may be opposite outer surface 310 and therefore not visible in the view of FIG. 3.

Outer surface 310 may be defined by a liquid-impermeable backsheet 314, and a liquid-permeable topsheet 316 defines body facing surface 312 and may be configured to be closest to the wearer during use. "Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. "Lamination" may be the technique of manufacturing a material in multiple layers, so that the composite material has benefits of all the combined layers, such as, for example, improved mechanical strength or durability, improved stability, lower permeability to water, and/or other properties. A laminate includes two or more layers of material(s) that are permanently assembled by heat, pressure, ultrasonic welding, or adhesive. Backsheets are typically liquid-impermeable and can include, for example, an inner liquid-impermeable film and an outer nonwoven backsheet that can be a nonwoven fabric. A "film" may be a membrane-like layer of material formed of one or more polymers, which does not have a form consisting predominately of a web-like structure of fibers and/or other fibers. In some absorbent articles, the backsheet can be breathable, for example, an inner liquid-impermeable film of the backsheet can include a breathable film. The terms "breathable," "breathable film," "breathable laminate" or "breathable outer cover material" or "breathable backsheet" refer to a film, laminate, or outer cover material having a water vapor transmission rate ("WVTR") of at least about 300 grams/meter²/24 hours. Breathable materials typically rely on molecular diffusion of vapor and are substantially liquid impermeable. "Nonwoven backsheet" may be a backing substrate layer in the outer cover; a nonwoven backsheet may be most often a nonwoven layer facing away from the wearer.

Absorbent article 300 includes back ear panels 318. First ends of back ear panels 318 are bonded to rear waist portion of 306 of chassis 302 and second ends of back ear panels 318 extend away from rear waist portion 306. "Bonded" refers to the joining, adhering, connecting, attaching, or the like, of two elements via adhesive(s), ultrasonic bond(s), and/or thermal bond(s). Two elements will be considered to be bonded together when they are bonded directly to one another or indirectly to one another, such as when each is directly bonded to intermediate elements. Back ear panels 318 are each formed of a stretchable elastic material, such as a nonwoven laminate, that permits adjustment in the width and tension of back ear panels 318 to vary the form and fit of absorbent article 300 when worn by a user.

Each back ear panel 318 includes a fastener tab 319 with a first end bonded to the corresponding back ear panel 318 and a second end that extends laterally outward from the corresponding back ear panel 318. Fastener tabs 319 can each include fastener portions, such as a hook material configured to interact with a corresponding loop material, an adhesive, or another type of fastener. Fastener tabs 319 are configured to be engaged by front waist portion 304, such as by front ear panels or a landing zone. Although back ear panels 318 and fastener tabs 319 are illustrated, in other implementations, absorbent article 300 may include youth training pants, adult incontinence pants, or the like, that do not include back ear panels 318 and fastener tabs 319 and may be always configured in a wearable configuration.

Absorbent article 300 also includes an absorbent core 320 disposed between topsheet 316 and backsheet 314. Absorbent core 320 may be configured to longitudinally extend along crotch portion 308. A longitudinal length of absorbent core 320 can be, for example, greater than or equal to, or between two of, 300, 330, 360, 420, 450, 480, 510, 540, 560, 570, or 600 millimeters (e.g., between 420 and 480 millimeters), as non-limiting examples. Absorbent core 320 can be coupled to crotch portion 308 and can, but need not, extend longitudinally along the entire length of crotch portion 308. For example, absorbent core 320 can have a longitudinal length at least, or between any two of, 10%, 20%, 30%, 40%, 50%, 60%, 70%, 80%, or 90% smaller than a length of crotch portion 308, and optionally be positioned closer to front waist portion 304 than to rear waist portion 306. Absorbent core 320 may include any material or combination of materials suitable for absorbing liquids, such as, for example, a laminate (e.g., a single layer laminae or a multi-layer laminate). The laminate may be formed from a nonwoven fabric or material. "Nonwoven" fabrics, according to an INDA (Association of the Nonwoven Fabrics Industry) definition, are broadly defined as sheet or web structures bonded together by entangling fiber or filaments, and by perforating films, mechanically, thermally, or chemically. Nonwoven fabrics are flat, porous sheets that are made directly from separate fibers or from molten plastic or plastic film. Nonwoven fabrics are not made by weaving or knitting and do not require converting the fibers to yarn. The basis weight of nonwoven fabrics is usually expressed as grams per square meter (gsm).

Absorbent core 320 also contains materials like super absorbent particles (SAP) and/or cellulosic fibers that are configured to absorb liquid in absorbent article 300. "Superabsorbent" or "superabsorbent material" or "SAP" refers to a water-swellable, water-insoluble organic or inorganic material capable, under the most favorable conditions, of absorbing at least about 15 times its weight in an aqueous solution containing 0.9 weight percent sodium chloride and, more desirably, at least about 30 times its weight in an aqueous solution containing 0.9 weight percent sodium chloride and, even more desirably, at least about 50 times its weight in an aqueous solution containing 0.9 weight percent sodium chloride. Illustrative superabsorbent polymer material suitable for use in absorbent core 320 can include any superabsorbent polymer particles known from superabsorbent literature, for example such as described in Modern Superabsorbent Polymer Technology, F. L. Buchholz, A. T. Graham, Wiley 1998. For example, the SAP particles may be spherical, spherical-like irregularly shaped particles, such as sausage shaped particles, or ellipsoid shaped particles of the kind typically obtained from inverse phase suspension polymerizations. The SAP particles can also be optionally agglomerated at least to some extent to form larger particles. In some implementations, the SAP particles can also have surface modifications, such as a partial or full surface coating or crosslinking, for example to increase the gel strength of the SAP particles.

The SAP materials can be natural, synthetic and modified natural polymers and materials. In addition, the SAP materials can be or may include organic compounds such as cross linked polymers. "Cross-linked" is a commonly understood term and refers to any approach for effectively rendering normally water-soluble materials substantially water insoluble, but swellable. Such polymers can include, for example, carboxymethylcellulose, alkali metal salts of polyacrylic acids, polyacrylamides, polyvinyl ethers, hydroxypropyl cellulose, polyvinyl morpholinone, polymers and copolymers of vinyl sulfonic acid, polyacrylates, polyacrylamides, polyvinyl pyridine and the like. Other suitable polymers include hydrolyzed acrylonitrile grafted starch, acrylic acid grafted starch, and isobutylene maleic anhydride copolymers, and mixtures thereof. Organic high-absorbency materials can include natural materials, such as agar, pectin, guar gum and peat moss. In addition to organic materials, superabsorbent materials may also include inorganic materials, such as absorbent clays and silica gels. Suitable examples of SAP include T9030, T9600, T9900, and Saviva polymers from BASF Corporation in Charlotte, North Carolina; and W211, W112A, W125, S125D, QX-W1482, QX-W1486, QX-W1504, and QX-W1505 from Nippon Shokubai Co. Ltd, N.A.I.I. in Houston, Texas; and AQUA KEEP SA50 II, SA55SX II, SA60N II, SA65S, HP500E, HP600, and HP 700E from Sumitomo Seika Chemicals Co., Ltd. in Osaka, Japan.

In some articles, the SAP can have a centrifuge retention capacity of 20-60 grams per gram (g/g), for example 30-50 g/g or 33-52 g/g, optionally between 33 and 38 g/g, or optionally between 44 and 48 g/g, as non-limiting examples. The SAP can have particle size distribution (PSD) with most or substantially all particles having a diameter between 150 micrometers (µm) and 850 µm. Preferably, all or substantially all of the SAP particles in at least one of the absorbent laminae have a diameter less than or equal to 500 µm to reduce the roughness of the absorbent laminae. For example, ones of the SAP particles in absorbent core 320 having a diameter greater than or equal to 500 µm can account for less than 10% (e.g., less than 3% or less than 0.2%) of the mass of the SAP particles in the core. An illustrative SAP suitable for absorbent core 320 may be HP500E from Sumitomo Seika Chemicals Co., Ltd. in Osaka, Japan. As used herein, particle diameter refers to the equivalent diameter of the particle if the particle is modelled as a sphere.

In some implementations, the SAP material of the absorbent laminae can be disposed within a matrix of adhesive material. Suitable adhesive material can include, for example, a thermoplastic hot-melt adhesive composition or a pressure-sensitive thermoplastic adhesive composition. For example, an absorbent lamina can include at least 90% (e.g., greater than 93% or 94%), by weight, SAP and less than or equal to 10% (e.g., less than 6% or 7%), by weight, adhesive. To illustrate, the SAP of the absorbent lamina can have a basis weight of at least 40 grams per square meter (gsm), such as, for example, greater than or equal to or between any two of 40, 50, 60, 70, 80, 90, 100 or more gsm (e.g., between 60 and 75 gsm).

Absorbent core 320 may include a single channel 322. Channel 322 may have one or more pathways that extend longitudinally along absorbent core 320. In the implementation illustrated in FIG. 3, channel 322 includes a single pathway that extends longitudinally along absorbent core 320. Channel 322 may be substantially free of absorbent material. As used herein, "substantially free" of absorbent materials means that channel 322 includes one of, or between any two of, 0%, 1%, 2%, 3%, 4%, 5%, 6%, 7%, 8%, 9%, or 10% absorbent materials, such as SAP or fibers. Channel 322 may be configured to provide a pathway for channeling fluids longitudinally through absorbent core 320. At least a portion of channel 322 may be disposed adjacent to a wearer's groin area. For example, at least a portion of channel 322 may be adjacent to a wearer's groin area when absorbent article 300 may be in a wearable configuration.

Absorbent core 320 may include an assembly that may be disposed between top core cover 326 and bottom core cover 327. In some implementations, top core cover 326 may be coupled to bottom core cover 327 at channel 322. For example, channel 322 may be substantially empty, allowing a layer above absorbent core 320 (e.g., top core cover 326) to be coupled to a layer below absorbent core 320 (e.g., bottom core cover 327). The absorbent core assembly (including top core cover 326 and bottom core cover 327) may be disposed between topsheet 316 and backsheet 314.

In some implementations, absorbent article 300 also includes a wetness indicator 324. Wetness indicator 324 may be coupled to backsheet 314. Wetness indicator 324 corresponds to at least a portion of channel 322. In the implementation illustrated in FIG. 3, wetness indicator 324 may be a line that extends longitudinally along absorbent core 320 and may be located above a middle portion of channel 322. Wetness indicator 324 may be configured to indicate a wetness of absorbent core 320. For example, wetness indicator 324 may be configured to change color when the amount of fluid contained by absorbent core 320 satisfies a threshold. As another example, wetness indicator 324 may be configured to display graphics or text on backsheet 314 when the amount of fluid contained by absorbent core 320 satisfies a threshold. Any type of wetness indicator may be used for wetness indicator 324.

In a particular implementation, an absorbent article (e.g., 300) includes a chassis (e.g., 302) having opposing front (e.g., 304) and rear (e.g., 306) waist portions and a crotch portion (e.g., 308) extending longitudinally between the front and rear waist portions. The crotch portion may be configured to conform about at least one of a wearer's groin area, perineum, and rear when the chassis may be configured in a wearable configuration. When in the wearable configuration, the front and rear waist portions preferably cooperate to encircle and define a waist opening, a left side of the chassis defines a first leg opening, and right side of the chassis defines a second leg opening. The absorbent article may also include an absorbent core (e.g., 320) extending longitudinally along the crotch portion. The absorbent core may include a channel (e.g., 322) having one or more pathways extending longitudinally along the absorbent core. The channel may be substantially free of material. The absorbent core may include an assembly disposed between a top core cover (e.g., 326) and a bottom core cover (e.g., 327). The absorbent core assembly may be disposed between a topsheet (e.g., 316) and a backsheet (e.g., 314).

Thus, FIG. 3 describes absorbent core 320 that may preferably include channel 322 that may be substantially free of absorbent material. Channel 322 helps to redirect fluids to other non-used portions of absorbent core 320 and helps to maintain stability of absorbent core 320 and assist with form-fitting to the wearer of absorbent article 300. For example, channel 322 provides a single pathway for channeling fluids longitudinally through the absorbent core. Channel 322 gives direct and immediate contact to wetness indicator 324 located central to channel 322. Additionally, bonding of bottom core cover 327 to top core cover 326 through channel 322 improves core integrity and helps hold absorbent materials in place during use of absorbent core 320, which improves fluid distribution and leakage protection of absorbent article 300. Also, bonding through the channel longitudinally through absorbent article 300 assists in contouring absorbent article 300 to the wearer, improving fit of absorbent article 300. Additionally, locating channel 322 at the crotch point of the wearer (e.g., adjacent to the wearer's crotch area) reduces or minimizes crotch width reduction of absorbent article 300 during use.

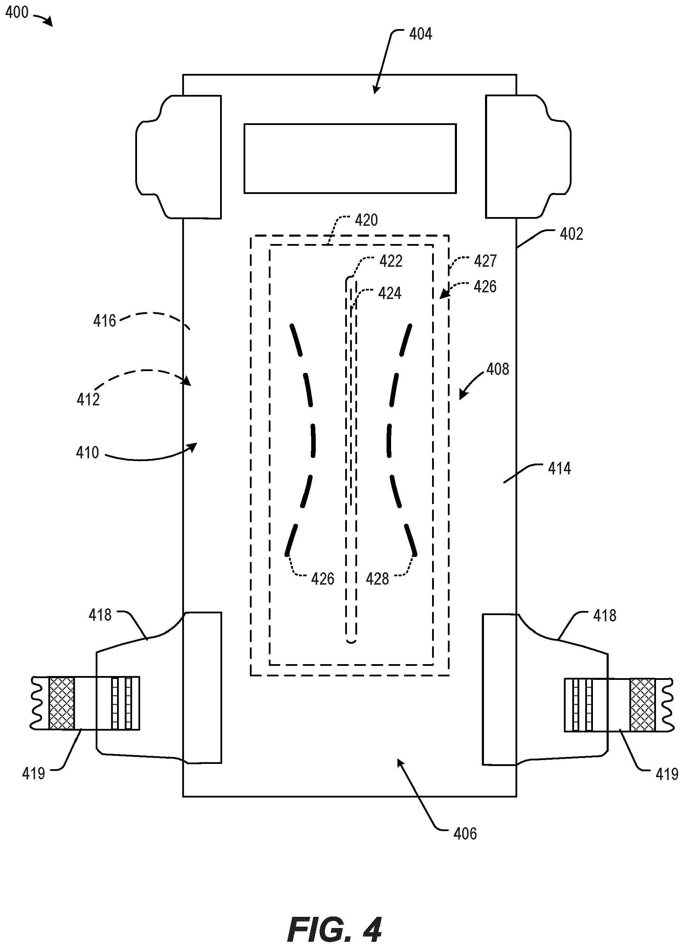

Referring to FIG. 4, an absorbent article 400 may be shown. Absorbent article 400 may be a disposable absorbent article, such as adult incontinence briefs, protective underwear, feminine hygiene pads, infant diapers, youth training pants, and the like, as illustrative, non-limiting examples.

Absorbent article 400 may preferably include a chassis 402 having a front waist portion 404, an opposing rear waist portion 406, and a crotch portion 408 extending longitudinally between front waist portion 404 and rear waist portion 406. Chassis 402 may further include an outer surface 410 configured to face away from a wearer during use of absorbent article 400, and an opposing body facing (or inner) surface 412 configured to face a wearer during use of absorbent article 400. Outer surface 410 may be defined by a liquid-impermeable backsheet 414, and a liquid-permeable topsheet 416 defines body facing surface 412 and may be configured to be closest to the wearer during use. Absorbent article 400 may preferably include back ear panels 418. First ends of back ear panels 418 are bonded to rear waist portion of 406 of chassis 402 and second ends of back ear panels 418 extend away from rear waist portion 406. Each back ear panel 418 may preferably include a fastener tab 419 with a first end bonded to the corresponding back ear panel 418 and a second end that extends laterally outward from the corresponding back ear panel 418. Fastener tabs 419 can each include fastener portions, such as a hook material configured to interact with a corresponding loop material, an adhesive, or another type of fastener. Fastener tabs 419 are configured to be engaged by front waist portion 404, such as by front ear panels or a landing zone. Although back ear panels 418 and fastener tabs 419 are illustrated, in other implementations, absorbent article 400 may include youth training pants, adult incontinence pants, or the like, that do not include back ear panels 418 and fastener tabs 419 and may be always configured in a wearable configuration.

Absorbent article 400 may also include an absorbent core 420 disposed between topsheet 416 and backsheet 414. Absorbent core 420 may be configured to longitudinally extend along crotch portion 408. A longitudinal length of absorbent core 420 can be, for example, greater than or equal to, or between two of, 300, 330, 360, 420, 450, 480, 510, 540, 560, 570, or 600 millimeters (e.g., between 420 and 480 millimeters), as non-limiting examples. Absorbent core 420 can be coupled to crotch portion 408 and can, but need not, extend longitudinally along the entire length of crotch portion 408. For example, absorbent core 420 can have a longitudinal length at least, or between any two of, 10%, 20%, 30%, 40%, 50%, 60%, 70%, 80%, or 90% smaller than a length of crotch portion 408, and optionally be positioned closer to front waist portion 404 than to rear waist portion 406.

Absorbent core 420 may include a single channel 422. Channel 422 may have one or more pathways that extend longitudinally along absorbent core 420. In the implementation illustrated in FIG. 4, channel 422 may preferably include a single pathway that extends longitudinally along absorbent core 420. Channel 422 may be substantially free of absorbent material. Channel 422 may be configured to provide a pathway for channeling fluids longitudinally through absorbent core 420. At least a portion of channel 422 may be disposed adjacent to a wearer's groin area. A wetness indicator 424 may be coupled to backsheet 414 and corresponds to channel 422, as described with reference to FIG. 3.

In some implementations, absorbent core 420 may preferably include embossing regions that extend at least partially longitudinally along absorbent core 420. For example, absorbent core 420 may include first embossing region 426 and second embossing region 428. Embossing regions 426-428 may be regions of absorbent core 420 that have been embossed and are now bonded together. The bond may be achieved through compression. Channel 422 may be disposed laterally between first embossing region 426 and second embossing region 428, as illustrated in FIG. 4. As shown in FIG. 4, embossing regions 426-428 may extend longitudinally along absorbent core 420, however, in the implementation illustrated in FIG. 4, embossing regions 426-428 are curved (e.g., arced) instead of straight. For example, first embossing region 426 and second embossing region 428 may be concave with reference to channel 422. In other implementations, embossing regions 426-428 may have other shapes or may be straight, similar to channel 422.

Thus, FIG. 4 describes absorbent core 420 that may preferably include channel 422 that may be substantially free of absorbent material and that may preferably include embossing regions 426-428. Embossing regions 426-428 can provide multiple benefits compared to conventional absorbent cores. For example, embossing regions 426-428 establish (e.g., increase) core integrity in absorbent core 420. Additionally, embossing regions 426-428 present a barrier to lateral fluid flow and redirect fluid within absorbent core 420 to regions to the front and rear of absorbent core 420 by means of capillary force in areas adjacent to embossing regions 426-428. Also, embossing regions 426-428 can further assist in establishing a preferred contoured shape of absorbent article 400 during use, which improves fit of absorbent article 400. In addition, the combination of channel 422 and embossing regions 426-428 provide the benefit of managing fluid flow within absorbent core 420. For example, channel 422 manages the initial fluid flow (e.g., surge) at the interface between absorbent core 420 and topsheet 416 (e.g., at the surface of absorbent core 420) and embossing regions 426-428 manage fluid flow after fluids have entered absorbent core 420.

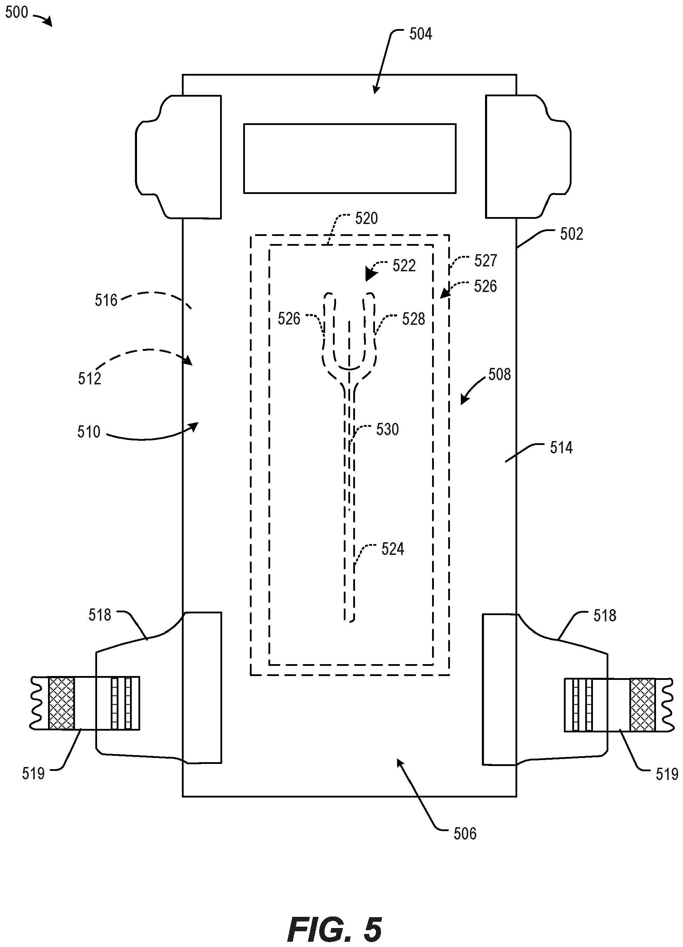

Referring to FIG. 5, an absorbent article 500 may be shown. Absorbent article 500 may be a disposable absorbent article, such as adult incontinence briefs, protective underwear, feminine hygiene pads, infant diapers, youth training pants, and the like, as illustrative, non-limiting examples.

Absorbent article 500 may preferably include a chassis 502 having a front waist portion 504, an opposing rear waist portion 506, and a crotch portion 508 extending longitudinally between front waist portion 504 and rear waist portion 506. Chassis 502 may further include an outer surface 510 configured to face away from a wearer during use of absorbent article 500, and an opposing body facing (or inner) surface 512 configured to face a wearer during use of absorbent article 500. Outer surface 510 may be defined by a liquid-impermeable backsheet 514, and a liquid-permeable topsheet 516 defines body facing surface 512 and may be configured to be closest to the wearer during use. Absorbent article 500 may preferably include back ear panels 518. First ends of back ear panels 518 are bonded to rear waist portion of 506 of chassis 502 and second ends of back ear panels 518 extend away from rear waist portion 506. Each back ear panel 518 may preferably include a fastener tab 519 with a first end bonded to the corresponding back ear panel 518 and a second end that extends laterally outward from the corresponding back ear panel 518. Fastener tabs 519 can each include fastener portions, such as a hook material configured to interact with a corresponding loop material, an adhesive, or another type of fastener. Fastener tabs 519 are configured to be engaged by front waist portion 504, such as by front ear panels or a landing zone. Although back ear panels 518 and fastener tabs 519 are illustrated, in other implementations, absorbent article 500 may include youth training pants, adult incontinence pants, or the like, that do not include back ear panels 518 and fastener tabs 519 and may be always configured in a wearable configuration.

Absorbent article 500 may also include an absorbent core 520 disposed between topsheet 516 and backsheet 514. Absorbent core 520 may be configured to longitudinally extend along crotch portion 508. A longitudinal length of absorbent core 520 can be, for example, greater than or equal to, or between two of, 300, 330, 360, 420, 450, 480, 510, 540, 560, 570, or 600 millimeters (e.g., between 420 and 480 millimeters), as non-limiting examples. Absorbent core 520 can be coupled to crotch portion 508 and can, but need not, extend longitudinally along the entire length of crotch portion 508. For example, absorbent core 520 can have a longitudinal length at least, or between any two of, 10%, 20%, 30%, 40%, 50%, 60%, 70%, 80%, or 90% smaller than a length of crotch portion 508, and optionally be positioned closer to front waist portion 504 than to rear waist portion 506.

Absorbent core 520 may include a single channel 522. Channel 522 may have one or more pathways that extend longitudinally along absorbent core 520. In the implementation illustrated in FIG. 5, channel 522 may preferably include three pathways: a first pathway 524 extending in a first direction, a second pathway 526 extending at least partially in a second direction that may be opposite to the first direction, and a third pathway 528 extending at least partially in the second direction. Second pathway 526 and third pathway 528 are referred to as extending at least partially in the second direction because portions of second pathway 526 and third pathway 528 extend in the second direction, and portions of the second pathway 526 and third pathway 528 extend in a transverse (e.g., lateral) direction. Thus, channel 522 may for a "Y" shape. Channel 522 may be substantially free of absorbent material. Channel 522 may be configured to provide a pathway for channeling fluids longitudinally and transversely (e.g., laterally) through absorbent core 520. At least a portion of channel 522 (e.g., a connection area between first pathway 524, second pathway 526, and third pathway 528) may be disposed adjacent to a wearer's groin area. A wetness indicator 530 may be coupled to backsheet 514 and correspond to channel 522, as described with reference to FIG. 3. Wetness indicator 530 may extend through at least a portion of first pathway 524 and the connection area between first pathway 524, second pathway 526, and third pathway 528.

Absorbent core 520 may include an assembly that may be disposed between top core cover 526 and bottom core cover 527. In some implementations, top core cover 526 may be coupled to bottom core cover 527 at channel 522. For example, channel 522 may be substantially empty, allowing a layer above absorbent core 520 (e.g., top core cover 526) to be coupled to a layer below absorbent core 520 (e.g., bottom core cover 527). The absorbent core assembly (including top core cover 526 and bottom core cover 527) may be disposed between topsheet 516 and backsheet 514.

In some implementations, absorbent core 520 may further include embossing regions, similar to embossing regions 426-428 of FIG. 4. For example, channel 522 may be disposed between a first embossing region and a second embossing region. The embossing regions may extend longitudinally along absorbent core 520.

In a particular implementation, an absorbent article (e.g., 500) may preferably include a chassis (e.g., 502) having opposing front (e.g., 504) and rear (e.g., 506) waist portions and a crotch portion (e.g., 508) extending longitudinally between the front and rear waist portions. The crotch portion may be configured to conform about at least one of a wearer's groin area, perineum, and rear when the chassis may be configured in a wearable configuration. When in the wearable configuration, the front and rear waist portions may preferably cooperate to encircle and define a waist opening, a left side of the chassis defines a first leg opening, and a right side of the chassis defines a second leg opening. The absorbent article may also include an absorbent core (e.g., 520) extending longitudinally along the crotch portion. The absorbent core may preferably include a channel (e.g., 522) having one or more pathways extending longitudinally along the absorbent core. The channel may be substantially free of material. The channel may preferably include a first pathway (e.g., 524) extending in a first direction, a second pathway (e.g., 526) extending at least partially in a second direction that may be opposite to the first direction, and a third pathway (e.g., 528) extending at least partially in the second direction. The absorbent core may include an assembly disposed between a top core cover (e.g., 526) and a bottom core cover (e.g., 527). The absorbent core assembly may be disposed between a topsheet (e.g., 516) and a backsheet (e.g., 514).

Thus, FIG. 5 describes absorbent core 520 that may preferably include channel 522 that may be substantially free of absorbent material. Channel 522 helps to redirect fluids to other non-used portions of absorbent core 520 and helps to maintain stability of absorbent core 520 and assist with form-fitting to the wearer of absorbent article 500. For example, channel 522 may redirect fluids in a longitudinal and transverse (e.g., lateral) directions throughout absorbent core 520. Additionally, channel 522 improves the contour of absorbent core 520 to a user, improving the fit of absorbent article 500.

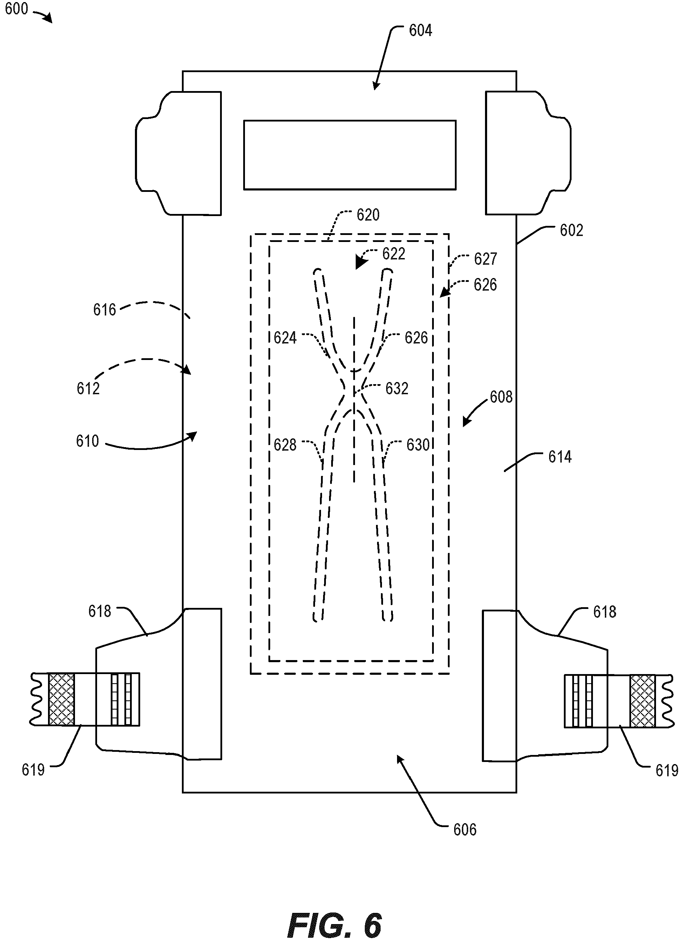

Referring to FIG. 6, an absorbent article 600 may be shown. Absorbent article 600 may be a disposable absorbent article, such as adult incontinence briefs, protective underwear, feminine hygiene pads, infant diapers, youth training pants, and the like, as illustrative, non-limiting examples.

Absorbent article 600 may preferably include a chassis 602 having a front waist portion 604, an opposing rear waist portion 606, and a crotch portion 608 extending longitudinally between front waist portion 604 and rear waist portion 606. Chassis 602 may further include an outer surface 610 configured to face away from a wearer during use of absorbent article 600, and an opposing body facing (or inner) surface 612 configured to face a wearer during use of absorbent article 600. Outer surface 610 may be defined by a liquid-impermeable backsheet 614, and a liquid-permeable topsheet 616 defines body facing surface 612 and may be configured to be closest to the wearer during use. Absorbent article 600 may preferably include back ear panels 618. First ends of back ear panels 618 are bonded to rear waist portion of 606 of chassis 602 and second ends of back ear panels 618 extend away from rear waist portion 606. Each back ear panel 618 may preferably include a fastener tab 619 with a first end bonded to the corresponding back ear panel 618 and a second end that extends laterally outward from the corresponding back ear panel 618. Fastener tabs 619 can each include fastener portions, such as a hook material configured to interact with a corresponding loop material, an adhesive, or another type of fastener. Fastener tabs 619 are configured to be engaged by front waist portion 604, such as by front ear panels or a landing zone. Although back ear panels 618 and fastener tabs 619 are illustrated, in other implementations, absorbent article 600 may include youth training pants, adult incontinence pants, or the like, that do not include back ear panels 618 and fastener tabs 619 and may be always configured in a wearable configuration.

Absorbent article 600 may also include an absorbent core 620 disposed between topsheet 616 and backsheet 614. Absorbent core 620 may be configured to longitudinally extend along crotch portion 608. A longitudinal length of absorbent core 620 can be, for example, greater than or equal to, or between two of, 300, 330, 360, 420, 450, 480, 510, 540, 560, 570, or 600 millimeters (e.g., between 420 and 480 millimeters), as non-limiting examples. Absorbent core 620 can be coupled to crotch portion 608 and can, but need not, extend longitudinally along the entire length of crotch portion 608. For example, absorbent core 620 can have a longitudinal length at least, or between any two of, 10%, 20%, 30%, 40%, 50%, 60%, 70%, 80%, or 90% smaller than a length of crotch portion 608, and optionally be positioned closer to front waist portion 604 than to rear waist portion 606.

Absorbent core 620 may include a single channel 622. Channel 622 may have one or more pathways that extend longitudinally along absorbent core 620. In the implementation illustrated in FIG. 6, channel 622 may preferably include four pathways: a first pathway 624, a second pathway 626, a third pathway 628, and a fourth pathway 630. First pathway 624 and second pathway 626 extend at least partially in a first direction, and third pathway 628 and fourth pathway 630 extend at least partially in a second direction that may be opposite to the first direction. Pathways 624-630 are referred to as extending at least partially in the first or second directions because portions of pathways 624-630 extend in the first or second direction, and portions of pathways 624-630 extend in a transverse (e.g., lateral) direction. Thus, channel 622 may form an "X" shape. Channel 622 may be substantially free of absorbent material. Channel 622 may be configured to provide pathways for channeling fluids longitudinally and transversely (e.g., laterally) through absorbent core 620. At least a portion of channel 622 (e.g., a connection area between first pathway 624, second pathway 626, third pathway 628, and fourth pathway 630) may be disposed adjacent to a wearer's groin area. A wetness indicator 632 may be coupled to backsheet 614 and corresponds to channel 622, as described with reference to FIG. 3. Wetness indicator 632 may extend through the connection area between first pathway 624, second pathway 626, third pathway 628, and fourth pathway 630.

Absorbent core 620 may include an assembly that may be disposed between top core cover 626 and bottom core cover 627. In some implementations, top core cover 626 may be coupled to bottom core cover 627 at channel 622. For example, channel 622 may be substantially empty, allowing a layer above absorbent core 620 (e.g., top core cover 626) to be coupled to a layer below absorbent core 620 (e.g., bottom core cover 627). The absorbent core assembly (including top core cover 626 and bottom core cover 627) may be disposed between topsheet 616 and backsheet 614.

In some implementations, absorbent core 620 may further include embossing regions, similar to embossing regions 426-428 of FIG. 4. For example, channel 622 may be disposed between a first embossing region and a second embossing region. The embossing regions may extend longitudinally along absorbent core 620.

In a particular implementation, an absorbent article (e.g., 600) may preferably include a chassis (e.g., 602) having opposing front (e.g., 604) and rear (e.g., 606) waist portions and a crotch portion (e.g., 608) extending longitudinally between the front and rear waist portions. The crotch portion may be configured to conform about at least one of a wearer's groin area, perineum, and rear when the chassis may be configured in a wearable configuration. When in the wearable configuration, the front and rear waist portions preferably cooperateto encircle and define a waist opening, a left side of the chassis defines a first leg opening, and a right side of the chassis defines a second leg opening. The absorbent article also includes an absorbent core (e.g., 620) extending longitudinally along the crotch portion. The absorbent core may preferably include a channel (e.g., 622) having one or more pathways extending longitudinally along the absorbent core. The channel may be substantially free of material. The channel may preferably include a first pathway (e.g., 624), a second pathway (e.g., 626), a third pathway (e.g., 628), and a fourth pathway (e.g., 630). The first pathway and the second pathway extend at least partially in a first direction, and the third pathway and the fourth pathway extend at least partially in a second direction that may be opposite to the first direction. The absorbent core may preferably include an assembly disposed between a top core cover (e.g., 626) and a bottom core cover (e.g., 627). The absorbent core assembly may be disposed between a topsheet (e.g., 616) and a backsheet (e.g., 614).

Thus, FIG. 6 describes absorbent core 620 that may preferably include channel 622 that may be substantially free of absorbent material. Channel 622 helps to redirect fluids to other non-used portions of absorbent core 620 and helps to maintain stability of absorbent core 620 and assist with form-fitting to the wearer of absorbent article 600. For example, channel 622 may redirect fluids in a longitudinal and transverse (e.g., lateral) directions throughout absorbent core 620. Additionally, channel 622 improves the contour of absorbent core 620 to a user, improving the fit of absorbent article 600.

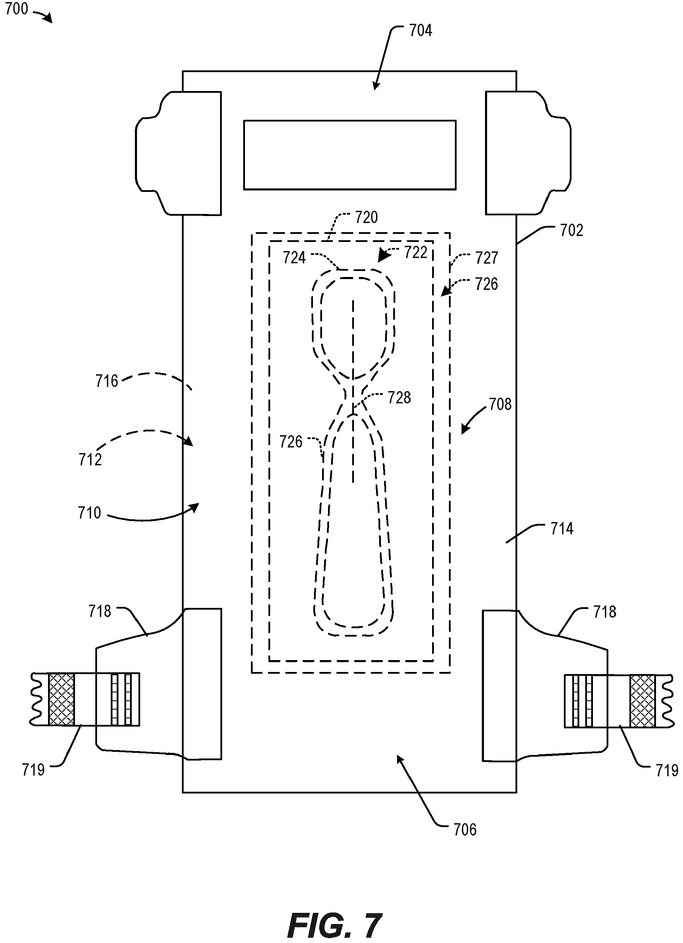

Referring to FIG. 7, an absorbent article 700 may be shown. Absorbent article 700 may be a disposable absorbent article, such as adult incontinence briefs, protective underwear, feminine hygiene pads, infant diapers, youth training pants, and the like, as illustrative, non-limiting examples.

Absorbent article 700 may preferably include a chassis 702 having a front waist portion 704, an opposing rear waist portion 706, and a crotch portion 708 extending longitudinally between front waist portion 704 and rear waist portion 706. Chassis 702 may further include an outer surface 710 configured to face away from a wearer during use of absorbent article 700, and an opposing body facing (or inner) surface 712 configured to face a wearer during use of absorbent article 700. Outer surface 710 may be defined by a liquid-impermeable backsheet 714, and a liquid-permeable topsheet 716 defines body facing surface 712 and may be configured to be closest to the wearer during use. Absorbent article 700 may preferably include back ear panels 718. First ends of back ear panels 718 are bonded to rear waist portion of 706 of chassis 702 and second ends of back ear panels 718 extend away from rear waist portion 706. Each back ear panel 718 may preferably include a fastener tab 719 with a first end bonded to the corresponding back ear panel 718 and a second end that extends laterally outward from the corresponding back ear panel 718. Fastener tabs 719 can each include fastener portions, such as a hook material configured to interact with a corresponding loop material, an adhesive, or another type of fastener. Fastener tabs 719 are configured to be engaged by front waist portion 704, such as by front ear panels or a landing zone. Although back ear panels 718 and fastener tabs 719 are illustrated, in other implementations, absorbent article 700 may include youth training pants, adult incontinence pants, or the like, that do not include back ear panels 718 and fastener tabs 719 and may be always configured in a wearable configuration.

Absorbent article 700 may also include an absorbent core 720 disposed between topsheet 716 and backsheet 714. Absorbent core 720 may be configured to longitudinally extend along crotch portion 708. A longitudinal length of absorbent core 720 can be, for example, greater than or equal to, or between two of, 300, 330, 360, 420, 450, 480, 510, 540, 560, 570, or 600 millimeters (e.g., between 420 and 480 millimeters), as non-limiting examples. Absorbent core 720 can be coupled to crotch portion 708 and can, but need not, extend longitudinally along the entire length of crotch portion 708. For example, absorbent core 720 can have a longitudinal length at least, or between any two of, 10%, 20%, 30%, 40%, 50%, 60%, 70%, 80%, or 90% smaller than a length of crotch portion 708, and optionally be positioned closer to front waist portion 704 than to rear waist portion 706.

Absorbent core 720 may include a single channel 722. Channel 722 may have one or more pathways that extend longitudinally along absorbent core 720. In the implementation illustrated in FIG. 7, channel 722 may preferably include two pathways: a first elliptical pathway 724 extending in a first direction and a second elliptical pathway 726 extending in a second direction that may be opposite to the first direction. Thus, channel 722 forms a "figure-8" shape. Channel 722 may be substantially free of absorbent material. Channel 722 may be configured to provide a pathway for channeling fluids longitudinally and transversely (e.g., laterally) through absorbent core 720. At least a portion of channel 722 (e.g., a connection area between first elliptical pathway 724 and second elliptical pathway 726) may be disposed adjacent to a wearer's groin area. A wetness indicator 728 may be coupled to backsheet 714 and corresponds to channel 722, as described with reference to FIG. 3. Wetness indicator 728 may extend through the connection area between first elliptical pathway 724 and second elliptical pathway 726.

Absorbent core 720 may include an assembly that may be disposed between top core cover 726 and bottom core cover 727. In some implementations, top core cover 726 may be coupled to bottom core cover 727 at channel 722. For example, channel 722 may be substantially empty, allowing a layer above absorbent core 720 (e.g., top core cover 726) to be coupled to a layer below absorbent core 720 (e.g., bottom core cover 727). The absorbent core assembly (including top core cover 726 and bottom core cover 727) may be disposed between topsheet 716 and backsheet 714.

In some implementations, absorbent core 720 may further include embossing regions, similar to embossing regions 426-428 of FIG. 4. For example, channel 722 may be disposed between a first embossing region and a second embossing region. The embossing regions may extend longitudinally along absorbent core 720.

In a particular implementation, an absorbent article (e.g., 700) may preferably include a chassis (e.g., 702) having opposing front (e.g., 704) and rear (e.g., 706) waist portions and a crotch portion (e.g., 708) extending longitudinally between the front and rear waist portions. The crotch portion may be configured to conform about at least one of a wearer's groin area, perineum, and rear when the chassis may be configured in a wearable configuration. When in the wearable configuration, the front and rear waist portions preferably cooperateto encircle and define a waist opening, the left side of the chassis defines a first leg opening, and the right side of the chassis defines a second leg opening. The absorbent article may also include an absorbent core (e.g., 720) extending longitudinally along the crotch portion. The absorbent core may preferably include a channel (e.g., 722) having one or more pathways extending longitudinally along the absorbent core. The channel may be substantially free of material. The channel may preferably include a first elliptical pathway (e.g., 724) extending in a first direction and a second elliptical pathway (e.g., 726) extending in a second direction that may be opposite to the first direction. The absorbent core may preferably include an assembly disposed between a top core cover (e.g., 726) and a bottom core cover (e.g., 727). The absorbent core assembly may be disposed between a topsheet (e.g., 716) and a backsheet (e.g., 714).

Thus, FIG. 7 describes absorbent core 720 that may preferably include channel 722 that may be substantially free of absorbent material. Channel 722 helps to redirect fluids to other non-used portions of absorbent core 720 and helps to maintain stability of absorbent core 720 and assist with form-fitting to the wearer of absorbent article 700. For example, channel 722 may redirect fluids in a longitudinal and transverse (e.g., lateral) directions throughout absorbent core 720. Additionally, channel 722 improves the contour of absorbent core 720 to a user, improving the fit of absorbent article 700.

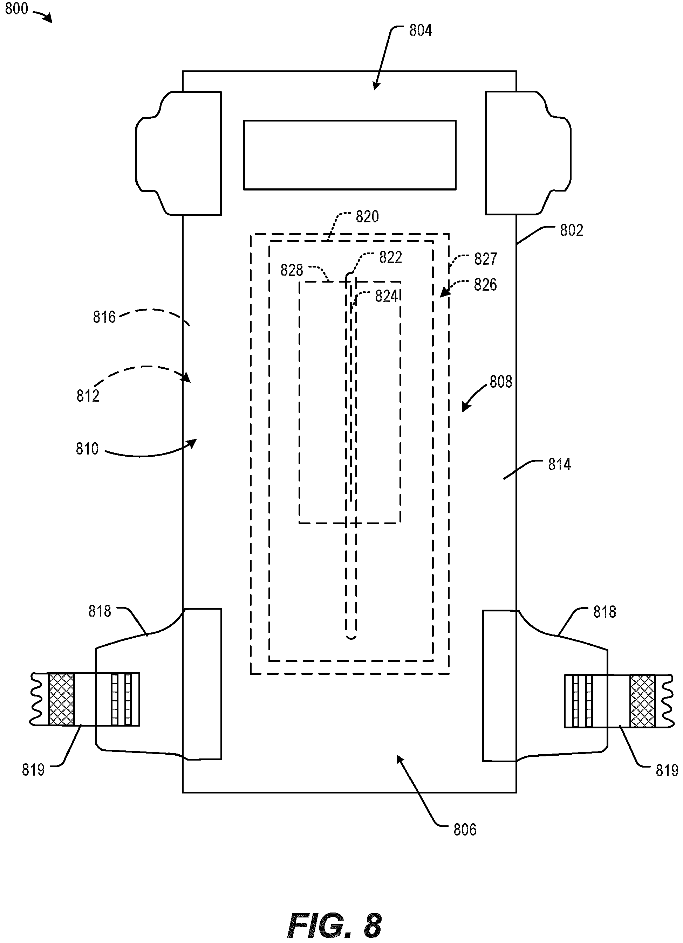

Referring to FIG. 8, an absorbent article 800 may be shown. Absorbent article 800 may be a disposable absorbent article, such as adult incontinence briefs, protective underwear, feminine hygiene pads, infant diapers, youth training pants, and the like, as illustrative, non-limiting examples.

Absorbent article 800 may preferably include a chassis 802 having a front waist portion 804, an opposing rear waist portion 806, and a crotch portion 808 extending longitudinally between front waist portion 804 and rear waist portion 806. Chassis 802 may further include an outer surface 810 configured to face away from a wearer during use of absorbent article 800, and an opposing body facing (or inner) surface 812 configured to face a wearer during use of absorbent article 800. Outer surface 810 may be defined by a liquid-impermeable backsheet 814, and a liquid-permeable topsheet 816 defines body facing surface 812 and may be configured to be closest to the wearer during use. Absorbent article 800 may preferably include back ear panels 818. First ends of back ear panels 818 are bonded to rear waist portion of 806 of chassis 802 and second ends of back ear panels 818 extend away from rear waist portion 806. Each back ear panel 818 may preferably include a fastener tab 819 with a first end bonded to the corresponding back ear panel 818 and a second end that extends laterally outward from the corresponding back ear panel 818. Fastener tabs 819 can each include fastener portions, such as a hook material configured to interact with a corresponding loop material, an adhesive, or another type of fastener. Fastener tabs 819 are configured to be engaged by front waist portion 804, such as by front ear panels or a landing zone. Although back ear panels 818 and fastener tabs 819 are illustrated, in other implementations, absorbent article 800 may include youth training pants, adult incontinence pants, or the like, that do not include back ear panels 818 and fastener tabs 819 and may be always configured in a wearable configuration.

Absorbent article 800 may also include an absorbent core 820 disposed between topsheet 816 and backsheet 814. Absorbent core 820 may be configured to longitudinally extend along crotch portion 808. A longitudinal length of absorbent core 820 can be, for example, greater than or equal to, or between two of, 300, 330, 360, 420, 450, 480, 510, 540, 560, 570, or 600 millimeters (e.g., between 420 and 480 millimeters), as non-limiting examples. Absorbent core 820 can be coupled to crotch portion 808 and can, but need not, extend longitudinally along the entire length of crotch portion 808. For example, absorbent core 820 can have a longitudinal length at least, or between any two of, 10%, 20%, 30%, 40%, 50%, 60%, 70%, 80%, or 90% smaller than a length of crotch portion 808, and optionally be positioned closer to front waist portion 804 than to rear waist portion 806.

Absorbent core 820 may include a single channel 822. Channel 822 may have one or more pathways that extend longitudinally along absorbent core 820. In the implementation illustrated in FIG. 8, channel 822 may include a single pathway that extends longitudinally along absorbent core 820. Channel 822 may be substantially free of absorbent material. Channel 822 may be configured to provide a pathway for channeling fluids longitudinally through absorbent core 820. At least a portion of channel 822 may be disposed adjacent to a wearer's groin area. A wetness indicator 824 may be coupled to backsheet 814 and corresponds to channel 822, as described with reference to FIG. 3.

Absorbent core 820 may include an assembly that may be disposed between top core cover 826 and bottom core cover 827. In some implementations, top core cover 826 may be coupled to bottom core cover 827 at channel 822. For example, channel 822 may be substantially empty, allowing a layer above absorbent core 820 (e.g., top core cover 826) to be coupled to a layer below absorbent core 820 (e.g., bottom core cover 827). The absorbent core assembly (including top core cover 826 and bottom core cover 827) may be disposed between topsheet 816 and backsheet 814.

In some implementations, absorbent core 820 may further include embossing regions, similar to embossing regions 426-428 of FIG. 4. For example, channel 822 may be disposed between a first embossing region and a second embossing region. The embossing regions may extend longitudinally along absorbent core 820.

Absorbent article 800 may further include an acquisition-distribution layer (ADL) 828. ADL 828 may preferably include a layer configured to absorb and distribute fluids from topsheet 816 to absorbent core 820. ADL 828 may be coupled to a top surface of absorbent core 820, such that ADL 828 may be disposed between the absorbent core assembly and topsheet 816 (e.g., below topsheet 816). In other implementations, other layers may be included between topsheet 816 and backsheet 814. For example, absorbent article 800 may include a back carrier sheet coupled to a bottom surface of a containment layer, and a top surface of the containment layer may be coupled to the bottom surface of the absorbent core assembly. Additionally, or alternatively, absorbent article 800 may include a dryness layer coupled in conjunction with (or in place of) ADL 828 to the top surface of the absorbent core and to topsheet 816.

The above specification and examples provide a complete description of the structure and use of illustrative implementations. Although certain examples have been described above with a certain degree of particularity, or with reference to one or more individual examples, those skilled in the art could make numerous alterations to the disclosed implementations without departing from the scope of this invention. As such, the various illustrative implementations of the methods and systems are not intended to be limited to the particular forms disclosed. Rather, they include all modifications and alternatives falling within the scope of the claims, and examples other than the one shown may include some or all of the features of the depicted example. For example, elements may be omitted or combined as a unitary structure, and/or connections may be substituted. Further, where appropriate, aspects of any of the examples described above may be combined with aspects of any of the other examples described to form further examples having comparable or different properties and/or functions, and addressing the same or different problems. Similarly, it will be understood that the benefits and advantages described above may relate to one embodiment or may relate to several implementations.

The claims are not intended to include, and should not be interpreted to include, means-plus- or step-plus-function limitations, unless such a limitation is explicitly recited in a given claim using the phrase(s) "means for" or "step for," respectively.

In the description "can", "may" and "preferably" each indicate that the corresponding features are optional and/or alternative features of preferred embodiments, i.e. these features can be present or omitted without departing from the invention. In particular, these features can be combined with any other non-contradictive feature.

## Claims

1. An absorbent article comprising:
a chassis having opposing front and rear waist portions and a crotch portion extending longitudinally between the front and rear waist portions, where the crotch portionis configured to conform about at least one of a wearer's groin area, perineum, and rear when the chassis is configured in a wearable configuration, where, when in the wearable configuration, the front and rear waist portions cooperate to encircle and define a waist opening, a left side of the chassis defines a first leg opening, and a right side of the chassis defines a second leg opening; and
an absorbent core extending longitudinally along the crotch portion ;
where the absorbent core comprises an assembly disposed between a top core cover and a bottom core cover; and
where the absorbent core assembly is disposed between a topsheet and a backsheet.
**characterized in that** the absorbent core comprises a channel having one or more pathways extending longitudinally along the absorbent core, the channel substantially free of absorbent material;

2. The absorbent article of claim 1, **characterized in that** the channel comprises a single channel.

3. The absorbent article of any of claims 1 or 2, **characterized in that** the single channel comprises a single pathway extending longitudinally along the absorbent core.

4. The absorbent article of any of claims 1-3, **characterized in that** the absorbent core further comprises embossing regions that extend at least partially longitudinally along the absorbent core.

5. The absorbent article of claim 4, **characterized in that** the channel is disposed laterally between a first embossing region of the embossing regions and a second embossing region of the embossing regions.

6. The absorbent article of claim 5, **characterized in that** the first embossing region and the second embossing region are concave with reference to the channel.

7. The absorbent article of any of claims 1-6, **characterized in that** the single channel comprises a first pathway extending in a first direction, a second pathway extending at least partially in a second direction that is opposite to the first direction, and a third pathway extending at least partially in the second direction.

8. The absorbent article of any of claims 1-7, **characterized in that** the single channel comprises a first pathway, a second pathway, a third pathway, and a fourth pathway, where the first pathway and the second pathway extend at least partially in a first direction, and where the third pathway and the fourth pathway extend at least partially in a second direction that is opposite to the first direction.

9. The absorbent article of any of claims 1-8, **characterized in that** the single channel comprises a first elliptical pathway that extends in a first direction and a second elliptical pathway that extends in a second direction that is opposite to the first direction.

10. The absorbent article of any of claims 1-9, **characterized in that** the absorbent article further comprises a wetness indicator coupled to the backsheet, the wetness indicator corresponding to at least a portion of the channel.

11. The absorbent article of any of claims 1-10, **characterized in that** at least a portion of the channel is disposed adjacent to the wearer's groin area and/or **in that** the top core cover is coupled to the bottom core cover at the channel.

12. The absorbent article of any of claims 1-11, further comprising an acquisition-distribution layer (ADL), the ADL positioned above the absorbent core assembly and below the topsheet.

13. The absorbent article of claim 12, further comprising a dryness layer positioned in conjunction with the ADL.

14. The absorbent article of any of claims 1-13, further comprising a containment layer positioned below the absorbent core assembly and above the backsheet.

15. The absorbent article of any one of claims 1 to 14, **characterized in that** the channel comprises:
a first pathway extending in a first direction, a second pathway extending at least partially in a second direction that is opposite to the first direction, and a third pathway extending at least partially in the second direction; and/or
a first pathway, a second pathway, a third pathway, and a fourth pathway, where the first pathway and the second pathway extend at least partially in a first direction, and where the third pathway and the fourth pathway extend at least partially in a second direction that is opposite to the first direction; and/or
a first elliptical pathway that extends in a first direction and a second elliptical pathway that extends in a second direction that is opposite to the first direction.
